# EUROPEAN PATENT APPLICATION

(11) **EP 3 278 804 A1**
(43) Date of publication of application: **07.02.2018**
(21) Application number: 16773230.4
(22) Date of filing: 01.04.2016
(51) Int. Cl.: A61K 31/568, A61K 9/06, A61P 27/02

(54) **DRY EYE THERAPEUTIC AGENT HAVING AS ACTIVE INGREDIENT NANDROLONE OR ESTER THEREOF OR METHENOLONE OR ESTER THEREOF**

(30) Priority: 03.04.2015 JP 2015076889
(71) Applicant: Santen Pharmaceutical Co., Ltd., Higashiyodogawa-ku Osaka-shi Osaka 533-8651 (JP)
(72) Inventor: ENDO, Kenichi, Ikoma-shi Nara 630-0101 (JP); FUJII, Shinobu, Ikoma-shi Nara 630-0101 (JP); OKI, Kenji, Ikoma-shi Nara 630-0101 (JP)
(74) Representative: Müller-Boré & Partner Patentanwälte PartG mbB
(86) International application number: PCT/JP2016/060927
(87) International publication number: WO 2016/159350

(57) **Abstract**

An object of the present invention is to provide a novel dry eye therapeutic agent that demonstrates superior dry eye therapeutic effects, and from the viewpoint of the drug administration burden of the patient, is capable of demonstrating a medicinal effect over a long period of time in lacrimal gland tissue after the administration thereof.

A dry eye therapeutic agent comprising nandrolone or an ester thereof or methenolone or an ester thereof is therapeutically effective on dry eye since it increases the amount of tear lipocalin (TL) in tears, and medicinal effects can be demonstrated over a long period of time in lacrimal gland tissue by administering a dry eye therapeutic agent comprising nandrolone or an ester thereof or methenolone or an ester thereof to eyelid skin.

## Description

### TECHNICAL FIELD

The present invention relates to a dry eye therapeutic agent comprising as an active ingredient nandrolone, an ester thereof, or a salt thereof, or methenolone, an ester thereof or a salt thereof.

### BACKGROUND ART

Although dry eye is a disease that initially exhibits merely unpleasant symptoms such as dryness of the eyes or eyes that feel sandy or gritty, as the condition worsens, it can cause considerable difficulties during the course of daily life. Although the pathology of dry eye is not completely clear, one of the major causes is thought to be defective formation of a stable tear film that covers the surface of the keratoconjunctiva caused by such factors as reduced lacrimation or increased tear evaporation.

Tear film break-up time (TFBUT) is known to be an indicator that is used to diagnose tear film stability and dry eye. In actuality, a TFBUT value of less than 5 seconds is one of the diagnostic criteria for dry eye. The behavior of a liquid film on a solid surface is affected by surface tension, and Non-Patent Document 1 indicates that there is a negative correlation between TFBUT and tear surface tension. Although the tear film contains numerous types of proteins secreted from the lacrimal glands as well as lipids and mucin derived from the Meibomian glands and goblet cells, Non-Patent Document 2 indicates that tear lipocalin (TL) is a particularly important component in tears in terms of decreases in tear surface tension and the integrity of the tear film, while Non-Patent Document 3 indicates that TL concentration in the tears of dry eye patients decreases and that there is a favorable correlation between TL and TFBUT. Namely, compounds that increase TL have a therapeutic effect on dry eye.

In addition, Non-Patent Document 4 indicates that lipophilin A-C complex present in human tears is decreased in evaporative dry eye patients, and that there is a significant correlation between TFBUT and subjective symptom score in the same manner as TL, while Non-Patent Document 5 indicates that lipophilin genes strongly expressed in rabbit tears consist of lipophilin CL/CL2 and lipophilin AL, and that these serve as important protein constituents of tears.

Examples of dry eye therapeutic agents used in Japan include sodium hyaluronate ophthalmic solution (Hyalein® ophthalmic solution 0. 1%), diguafosol sodium ophthalmic solution (Diquas® ophthalmic solution 3%), and rebamipide ophthalmic solution (Mucosta® ophthalmic suspension UD 2%). Although instillation for about 4 to 6 days is required to obtain therapeutic effects with these ophthalmic solutions, there are dry eye patients for whom adequate therapeutic effects are unable to be obtained even with these ophthalmic solutions, and the development of a new dry eye therapeutic agent is therefore still awaited.

Although examples of methenolone esters used in Japan include methenolone acetate and methenolone enanthate, these are used to treat conditions such as osteoporosis in the form of oral preparations and intramuscular injections, respectively. However, methenolone or esters thereof have not been used to treat eye diseases, and the effects demonstrated by methenolone or esters thereof in the lacrimal glands have not yet to be determined. In addition, nandrolone has been used in Japan in the form of nandrolone decanoate for the treatment of osteoporosis and the like in the same manner as methenolone. However, nandrolone or esters thereof have also not been used to treat eye diseases, and the effects demonstrated by nandrolone or esters thereof in the lacrimal glands have also not yet to be determined.

### [Prior Art Documents]

### [Non-Patent Documents]

[Non-Patent Document 1] Current Eye Research, 8, 507-515 (1989)
[Non-Patent Document 2] Biochimica et Biophysica Acta, 1482, 241-248 (2000)
[Non-Patent Document 3] Molecular Vision, 19, 1247-1257 (2013)
[Non-Patent Document 4] Eye, 24, 1396-1402 (2010)
[Non-Patent Document 5] Comparative Biochemistry and Physiology, Part B 138, 111-117 (2004)

### DISCLOSURE OF THE INVENTION

### [Problems to be Solved by the Invention]

An object of the present invention is to provide a novel dry eye therapeutic agent that demonstrates superior dry eye therapeutic effects, and from the viewpoint of the drug administration burden of the patient, is capable of demonstrating a medicinal effect over a long period of time in lacrimal gland tissue after the administration thereof.

### [Means for Solving the Problems]

As a result of conducting extensive research to seek out a novel dry eye therapeutic agent, the inventors of the present invention found that, when nandrolone decanoate or methenolone acetate was administered to the eyelid skin of dacryocystectomized rabbits, TL levels in tears increased, thereby leading to completion of the present invention.

Namely, the present invention relates to that indicated below.

(1) A dry eye therapeutic agent comprising nandrolone, an ester thereof or a salt thereof, or methenolone, an ester thereof or a salt thereof as an active ingredient.
(2) The dry eye therapeutic agent described in (1), which is characterized by being administered to eyelid skin.
(3) The dry eye therapeutic agent described in (1) or (2), which comprises nandrolone decanoate as an active ingredient.
(4) The dry eye therapeutic agent described in (1) or (2), which comprises methenolone acetate as an active ingredient
(5) The dry eye therapeutic agent described in (3) or (4), which comprises nandrolone decanoate or methenolone acetate at a concentration of 0.1 to 10 %(w/w) as an active ingredient.
(6) The dry eye therapeutic agent described in (3) or (4), which comprises nandrolone decanoate or methenolone acetate at a concentration of 1 to 5 %(w/w) as an active ingredient.
(7) The dry eye therapeutic agent described in any one of (1) to (6), which is characterized by being administered once a day to eyelid skin.
(8) The dry eye therapeutic agent described in any one of (1) to (7), wherein the administration form is an ointment or cream.
(9) The dry eye therapeutic agent described in any one of (1) to (7), wherein the administration form is a patch.
(10) A composition for secretory promotion of secretions from lacrimal glands, comprising nandrolone, an ester thereof or a salt thereof, or methenolone, an ester thereof or a salt thereof as an active ingredient.
(11) The composition described in (10), which is characterized by being administered to eyelid skin.
(12) The composition described in (10) or (11), which comprises nandrolone decanoate as an active ingredient.
(13) The composition described in (10) or (11), which comprises methenolone acetate as an active ingredient.
(14) The composition described in (12) or (13), which comprises nandrolone decanoate or methenolone acetate at a concentration of 0.1 to 10 %(w/w) as an active ingredient.
(15) The composition described in (12) or (13), which comprises nandrolone decanoate or methenolone acetate at a concentration of 1 to 5 %(w/w) as an active ingredient.
(16) The composition described in any one of (10) to (15), which is characterized by being administered once a day to eyelid skin.
(17) The composition described in any one of (10) to (16), wherein the administration form is an ointment or cream.
(18) The composition described in any one of (10) to (16), wherein the administration form is a patch.

The present invention also relates to that indicated below.
(19) A pharmaceutical composition for treating dry eye comprising nandrolone, an ester thereof or a salt thereof or methenolone, an ester thereof or a salt thereof, and a pharmaceutically acceptable additive.
(20) The pharmaceutical composition described in (19), which is characterized by being administered to eyelid skin.
(21) The pharmaceutical composition described in (19) or (20), which compirses nandrolone decanoate as an active ingredient.
(22) The pharmaceutical composition described in (19) or (20), which compirses methenolone acetate as an active ingredient.
(23) The pharmaceutical composition described in (21) or (22), which compirses nandrolone decanoate or methenolone acetate at a concentration of 0.1 to 10 %(w/w) as an active ingredient.
(24) The pharmaceutical composition described in (21) or (22), which compirses nandrolone decanoate or methenolone acetate at a concentration of 1 to 5 %(w/w) as an active ingredient.
(25) The pharmaceutical composition described in any one of (19) to (24), which is characterized by being administered once a day to eyelid skin.
(26) The pharmaceutical composition described in any one of (19) to (25), wherein the administration form is an ointment or cream.
(27) The pharmaceutical composition described in any one of (19) to (25), wherein the administration form is a patch.
(28) A use of nandrolone, an ester thereof or a salt thereof, or methenolone, an ester thereof or a salt thereof, for the manufacture of a medicament for treating dry eye.
(29) The use described in (28), characterized in that the medicament is administered to eyelid skin.
(30) The use described in (28) or (29), wherein the medicament contains nandrolone decanoate as an active ingredient.
(31) The use described in (28) or (29), wherein the medicament contains methenolone acetate as an active ingredient.
(32) The use described in (30) or (31), wherein the medicament contains nandrolone decanoate or methenolone acetate at a concentration of 0.1 to 10 %(w/w) as an active ingredient.
(33) The use described in (30) or (31), wherein the medicament contains nandrolone decanoate or methenolone acetate at a concentration of 1 to 5 %(w/w) as an active ingredient.
(34) The use described in any one of (28) to (33), characterized in that the medicament is administered once a day to eyelid skin.
(35) The use described in any one of (28) to (34), wherein the administration form of the medicament is an ointment or cream.
(36) The use described in any one of (28) to (34), wherein the administration form of the medicament is a patch.
(37) Nandrolone, an ester thereof, or a salt thereof, or methenolone, an ester thereof or a salt thereof, for use in the treatment of dry eye.
(38) The nandrolone, an ester thereof or a salt thereof, or methenolone, an ester thereof or a salt thereof, described in (37), which is characterized by being administered to eyelid skin.
(39) The nandrolone, an ester thereof or a salt thereof described in (37) or (38), which is characterized by being administered in the form of nandrolone decanoate.
(40) The methenolone, an ester thereof or a salt thereof described in (37) or (38), which is characterized by being administered in the form of methenolone acetate.
(41) The nandrolone, an ester thereof or a salt thereof, or methenolone, an ester thereof or a salt thereof, described in (39) or (40), characterized in that nandrolone decanoate or methenolone acetate is administered at a concentration of 0.1 to 10 %(w/w).
(42) The nandrolone, an ester thereof or a salt thereof, or methenolone, an ester thereof or a salt thereof, described in (39) or (40), characterized in that nandrolone decanoate or methenolone acetate is administered at a concentration of 1 to 5 %(w/w).
(43) The nandrolone, an ester thereof or a salt thereof, or methenolone, an ester thereof or a salt thereof, described in any one of (37) to (42), which is characterized by being administered once a day to eyelid skin.
(44) The nandrolone, an ester thereof or a salt thereof, or methenolone, an ester thereof or a salt thereof, described in any one of (37) to (43), characterized in that the administration form is an ointment or cream.
(45) The nandrolone, an ester thereof or a salt thereof, or methenolone, an ester thereof or a salt thereof, described in any one of (37) to (43), characterized in that the administration form is a patch.
(46) A method for the treatment of dry eye, comprising administering a therapeutically effective amount of nandrolone, an ester thereof or a salt thereof, or methenolone, an ester thereof or a salt thereof, to a subject.
(47) The method for the treatment described in (46), characterized in that the administration is to eyelid skin.
(48) The method for the treatment described in (46) or (47), characterized in that nandrolone decanoate is administered.
(49) The method for the treatment described in (46) or (47), characterized in that methenolone acetate is administered.
(50) The method for the treatment described in (48) or (49), characterized in that nandrolone decanoate or methenolone acetate is administered at a concentration of 0.1 to 10 %(w/w).
(51) The method for the treatment described in (48) or (49), characterized nandrolone decanoate or methenolone acetate is administered at a concentration of 1 to 5 %(w/w).
(52) The method for the treatment described in any one of (46) to (51), characterized in that the administration is once a day to eyelid skin.
(53) The method for the treatment described in any one of (46) to (52), wherein the administration form is an ointment or cream.
(54) The method for the treatment described in any one of (46) to (52), wherein the administration form is a patch.
(55) A method for secretory promotion of secretions from lacrimal glands, comprising administering an effective amount of nandrolone, an ester thereof or a salt thereof, or methenolone, an ester thereof or a salt thereof, to a subject.
(56) The method described in (55), characterized in that the administration is to eyelid skin.
(57) The method described in (55) or (56), characterized in that nandrolone decanoate is administered.
(58) The method described in (55) or (56), characterized in that methenolone acetate is administered.
(59) The method described in (57) or (58), characterized in that nandrolone decanoate or methenolone acetate is administered at a concentration of 0.1 to 10 %(w/w).
(60) The method described in (57) or (58), characterized in that nandrolone decanoate or methenolone acetate is administered at a concentration of 1 to 5 %(w/w).
(61) The method described in any one of (55) to (60), characterized in that the administration is once a day to eyelid skin.
(62) The method described in any one of (55) to (61), wherein the administration form is an ointment or cream.
(63) The method described in any one of (55) to (61), wherein the administration form is a patch.

### [Effects of the Invention]

A dry eye therapeutic agent comprising nandrolone or an ester thereof or methenolone or an ester thereof is therapeutically effective on dry eye since it increases the amount of TL in tears, and medicinal effects can be demonstrated over a long period of time in lacrimal gland tissue by administering a dry eye therapeutic agent comprising nandrolone or an ester thereof or methenolone or an ester thereof to eyelid skin.

### BRIEF DESCRIPTION OF THE DRAWINGS

FIG. 1 is a graph indicating the drug concentration in lacrimal gland tissue after administration of methenolone acetate to eyelid skin.
FIG. 2 is a graph indicating drug concentrations in lacrimal gland tissue after administration of an ointment containing nandrolone decanoate by applying to eyelid skin, and after administration of an ophthalmic solution containing nandrolone decanoate by instillation.
FIG. 3 is a graph indicating gene expression levels of lipophilin CL-2 (L-CL2) after having allowed nandrolone decanoate (ND) and methenolone acetate (MA) to act on lacrimal gland tissue cells.

### BEST MODE FOR CARRYING OUT THE INVENTION

Nandrolone is a compound (17β-hydroxy-19-nor-4-androsten-3-one) represented by the formula indicated below.

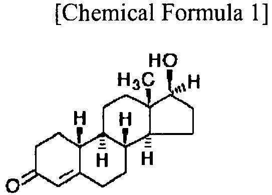

There are no particular limitations on esters of nandrolone provided that they are pharmaceutically acceptable nandrolone esters, and consist of nandrolone in which an ester bond has been formed by condensation of the hydroxyl group present in nandrolone with an organic acid or inorganic acid. Preferable examples of organic acids that condense with the hydroxyl group present in nandrolone include optionally branched monoalkyl carboxylic acids having 1 to 12 carbon atoms such as formic acid, acetic acid, propionic acid, butyric acid, isobutyric acid, valeric acid, isovaleric acid, pivalic acid, enanthic acid or decanoic acid, optionally branched alkyl dicarboxylic acids having 2 to 10 carbon atoms such as maleic acid, succinic acid or tartaric acid, optionally branched alkyl tricarboxylic acids having 3 to 10 carbon atoms such as citric acid, optionally branched alkyl sulfonic acids having 1 to 6 carbon atoms such as methanesulfonic acid, or phenyl sulfonic acids having 6 to 10 carbon atoms such as p-toluenesulfonic acid. Examples of inorganic acids that condense with the hydroxyl group present in nandrolone include boric acid and phosphoric acid. The nandrolone ester in the present invention is more preferably an ester formed with a monoalkyl carboxylic acid having 1 to 12 carbon atoms, and particularly preferably nandrolone decanoate.

Nandrolone decanoate is a compound (17β-decanoyloxy-19-nor-4-androsten-3-one) represented by the formula indicated below.

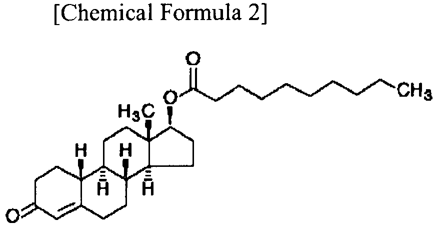

Methenolone is a compound (17β-hydroxy-1-methyl-5α-androst-1-en-3-one) represented by the formula indicated below.

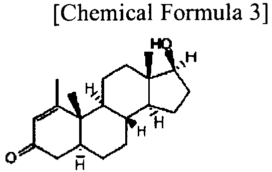

There are no particular limitations on esters of methenolone provided that they are pharmaceutically acceptable methenolone esters, and consist of methenolone in which an ester bond has been formed by condensation of the hydroxyl group present in methenolone with an organic acid or inorganic acid. Preferable examples of organic acids that condense with the hydroxyl group present in methenolone include optionally branched monoalkyl carboxylic acids having 1 to 12 carbon atoms such as formic acid, acetic acid, propionic acid, butyric acid, isobutyric acid, valeric acid, isovaleric acid, pivalic acid, enanthic acid or decanoic acid, optionally branched alkyl dicarboxylic acids having 2 to 10 carbon atoms such as maleic acid, succinic acid or tartaric acid, optionally branched alkyl tricarboxylic acids having 3 to 10 carbon atoms such as citric acid, optionally branched alkyl sulfonic acids having 1 to 6 carbon atoms such as methanesulfonic acid, or phenyl sulfonic acids having 6 to 10 carbon atoms such as p-toluenesulfonic acid. Examples of inorganic acids that condense with the hydroxyl group present in methenolone include boric acid and phosphoric acid. The methenolone ester in the present invention is more preferably an ester formed with a monoalkyl carboxylic acid having 1 to 12 carbon atoms, and particularly preferably methenolone acetate.

Methenolone acetate is a compound (17β-acetyloxy-1-methyl-5α-androst-1-en-3-one) represented by the formula indicated below.

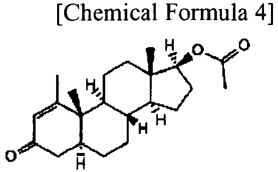

Methenolone enanthate is a compound (17β-(heptanoyloxy)-1-methyl-5α-androst-1-en-3-one) represented by the formula indicated below.

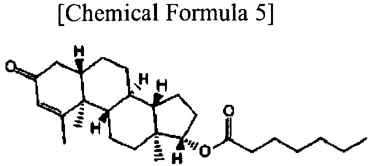

Nandrolone and esters thereof or methenolone and esters thereof can be produced in accordance with ordinary methods used in the field of synthetic organic chemistry. Furthermore, as was previously explained in the section describing the background art, oral preparations of methenolone acetate and intramuscular injection preparations of methenolone enanthate are available commercially as therapeutic agents for treating osteoporosis and the like.

There are no particular limitations on the salts of nandrolone or esters thereof or on the salts of methenolone or esters thereof provided that they are pharmaceutically acceptable salts, and examples thereof include salts formed with inorganic acids such as hydrochloric acid, hydrobromic acid, hydroiodic acid, nitric acid, sulfuric acid or phosphoric acid; salts formed with organic acids such as acetic acid, fumaric acid, maleic acid, succinic acid, citric acid, tartaric acid, adipic acid, gluconic acid, glucoheptonic acid, glucuronic acid, terephthalic acid, methanesulfonic acid, lactic acid, hippuric acid, 1,2-ethanedisulfonic acid, isethionic acid, lactobionic acid, oleic acid, pamoic acid, polygalacturonic acid, stearic acid, tannic acid, trifluoromethanesulfonic acid, benzenesulfonic acid, p-toluenesulfonic acid, lauryl sulfate, methyl sulfate, naphthalenesulfonic acid or sulfosalicylic acid; quaternary ammonium salts formed with methyl bromide or methyl iodide; salts formed with halogen ions such as bromine ions, chlorine ions or iodine ions; salts formed with alkaline metals such as lithium, sodium or potassium; salts formed with alkaline earth metals such as calcium or magnesium; metal salts formed with iron or zinc; salts formed with ammonia; and, salts formed with organic amines such as triethylenediamine, 2-aminoethanol, 2,2-iminobis(ethanol), 1-deoxy-1 -(methylamino)-2-D-sorbitol, 2-amino-2-(hydroxymethyl)-1 ,3-propanediol, procaine or N,N-bis(phenylmethyl)-1,2-ethanediamine.

In the present invention, salts of nandrolone or esters thereof or salts of methenolone or esters thereof also include hydrates and solvates of nandrolone or esters thereof or methenolone or esters thereof.

In the case nandrolone, an ester thereof or a salt thereof, or methenolone, an ester thereof or a salt thereof, has geometric isomers or optical isomers, those isomers or salts thereof are included within the scope of the present invention. In addition, in the case nandrolone, an ester thereof or a salt thereof, or methenolone, an ester thereof or a salt thereof, has proton tautomerism, those tautomers or salts thereof are also included within the scope of the present invention.

In the case nandrolone, an ester thereof or a salt thereof, or methenolone, an ester thereof or a salt thereof, has crystal polymorphs and crystal polymorph groups (polymorphic crystal systems), these crystal polymorphs and crystal polymorph groups (polymorphic crystal systems) are included within the scope of the present invention. Here, a crystal polymorph group (polymorphic crystal system) refers to individual crystal forms at each stage in the case the crystal form undergoes a change in manufacture of crystal, crystallization, storage or other conditions or state (with state also including the state of having been formulated into a preparation), as well as the entire process thereof.

Although the present dry eye therapeutic agent can also contain an active ingredient other than nandrolone, an ester thereof or a salt thereof or methenolone, an ester thereof or a salt thereof, the present dry eye therapeutic agent preferably contains nandrolone, an ester thereof or a salt thereof or methenolone, an ester thereof or a salt thereof as the sole active ingredient.

In the present invention, "dry eye" is defined as a "chronic disease by a variety fact of the tears and keratoconjunctival epithelium that is associated with eye discomfort and visual disorders". Dry eye in the present invention includes keratoconjunctivitis sicca (KCS), and includes both types of dry eye consisting of hyposecretive dry eye and evaporative dry eye.

Hyposecretive dry eye is categorized into Sjögren's syndrome-associated dry eye and non-Sjögren's syndrome dry eye. Examples of non-Sjogren's syndrome dry eye include dry eye associated with lacrimal gland diseases such as congenital alacrima, sarcoidosis or graft versus host disease (GVHD) afer bone marrow transplant, dry eye associated with tear duct obstruction caused by ocular pemphigoid, Stevens-Johnson syndrome or trachoma, and dry eye associated with decreased reflex secretion caused by diabetes or corneal refractive surgery (laser-assisted in situ keratomileusis, LASIK).

In addition, examples of evaporative dry eye include dry eye associated with reduced oily layer caused by Meibomian gland dysfunction or blepharitis, dry eye associated with nictation failure or eyelid closure failure caused by exophthalmos or lagophthalmos, dry eye associated with deterioration of lacrimal stability due to contact lens wearing, dry eye associated with decreased secretion of mucin from goblet cells, and dry eye associated with video display terminal (VDT) work.

"Dry eye therapy" in the present invention is defined as improvement of pathological symptoms and/or findings associated with dry eye, and not only refers to improvement of subjective symptoms such as eye dryness, eye discomfort, eye fatigue, sensation of heavy eyes, photophobia, eye pain or blurred vision associated with dry eyes, but also includes improvement of congestion or keratoconjunctival epithelial disorders associated with dry eye. Moreover, dry eye therapy also includes promotion and improvement of secretion of secretions from the lacrimal glands to a degree that permits favorable formation of tear film. Although examples of secretions from lacrimal glands include tears, these particularly include TL contained in tears.

In particular, the present dry eye therapeutic agent demonstrates therapeutic efficacy on dry eye by increasing the amount of TL present in tears. Thus, the present invention provides a composition comprising nandrolone or methenolone and the like as an active ingredient that increases the amount of TL present in tears. Furthermore, the amount of TL present in tears can be easily measured by enzyme-linked immunosorbent assay (ELISA).

The present dry eye therapeutic agent may be administered in a form that allows the nandrolone, an ester thereof or a salt thereof or methenolone, an ester thereof or a salt thereof to reach the lacrimal glands. For example, nandrolone, an ester thereof or a salt thereof, or methenolone, an ester thereof or a salt thereof may be administered followed by being metabolized or decomposed in the body, enabling it to reach the lacrimal glands in the form of nandrolone or methenolone. Although examples of administration forms (administration routes) of the present dry eye therapeutic agent include dermal administration, instillation (including instillation of an eye ointment), subconjunctival administration, conjunctival sac administration, sub-Tenon's capsule administration and oral administration, dermal administration is preferable, and administration to eyelid skin is particularly preferable.

In the present invention, eyelid skin includes the upper eyelid, lower eyelid and skin in the vicinity thereof, and preferably refers to skin of the upper eyelid.

Furthermore, in the case of administering the present dry eye therapeutic agent to eyelid skin, the present dry eye therapeutic agent can comprise methenolone acetate or nandrolone decanoate as an active ingredient, and preferably comprises nandrolone decanoate as an active ingredient in particular.

An additive can be used as necessary in the present dry eye therapeutic agent, and examples of additives that can be added include bases, surfactants, buffering agents, isotonic agents, stabilizers, preservatives, antioxidants, polymers and solvents.

There are no particular limitations on the administration form of the present dry eye therapeutic agent provided it is a drug form that allows nandrolone, an ester thereof or a salt thereof, or methenolone, an ester thereof or a salt thereof to reach the lacrimal glands. Examples of administration forms of the present dry eye therapeutic agent include a cream preparation, ointment preparation, patch preparation, liquid preparation, lotion preparation, ophthalmic preparation, injection preparation, gel preparation and insert preparation, and a cream, ointment and patch preparation are particularly preferable as drug forms suitable for administration to eyelid skin. Furthermore, these drug forms can be prepared by using ordinary techniques commonly used in the relevant field. Moreover, the present dry eye therapeutic agent can also be used as a preparation for intraocular implants or a preparation employed in a drug delivery system (DDS) such as microspheres in addition to these preparations.

An ointment preparation can be prepared in the form of an oily ointment by using mineral-based white vaseline or liquid paraffin, or an animal or plant-based castor oil, olive oil, lanolin or beeswax for the base. In addition, an aqueous ointment can be prepared by using macrogol, for example.

A cream preparation can be prepared by using a mineral-based product (such as white vaseline or liquid paraffin), animal or plant-based product (such as castor oil, olive oil, lanolin or beeswax), fatty acid or fatty acid ester (such as palmitic acid, stearic acid, oleic acid or isopropyl myristate), medium-chain fatty acid triglyceride (such as Miglyol 812), higher alcohol (such as stearyl alcohol) or polyvalent alcohol (such as glycerin, propylene glycol, macrogol or sorbitol) as base, by using a polyvalent alcohol fatty acid ester (such as a glycerin fatty acid ester), ethylene oxide addition-type nonionic surfactant (such as Cremophor EL), anionic surfactant (such as a sodium fatty acid salt or sodium alkyl sulfonate), cationic surfactant (such as an alkyl trimethyl ammonium salt), amphoteric surfactant (such as sodium alkylamino fatty acid) or lecithin derivative as surfactant, and by using a thickening polymer such as polycarbophil or carboxyvinyl polymer as thickener, and by additionally using a preservative (such as methyl paraben or propyl paraben), isotonic agent (such as sodium chloride or concentrated glycerin) and/or an absorption promoter (such as ethanol) or pH adjuster (such as sodium hydroxide, potassium hydroxide, triethanolamine or sodium citrate) as necessary.

A patch preparation can be prepared by using an adhesive (which may also contain an additive such as a tackifying resin, crosslinking agent, plasticizer, surfactant or antioxidant) and a support, and by additionally using a preservative and/or absorption promoter as necessary. Furthermore, an adhesive tape with low peeling force as a function thereof is used for administration to eyelid skin.

Examples of adhesives used in a patch include acrylic-based, silicon-based and rubber-based adhesives. Examples of tackifying resins added in adhesives include rosin-based, terpene-based and petroleum resin-based resins. Polyisocyanate-based crosslinking agents are mainly used as crosslinking agents. Examples of the support include a non-woven fabric, film and sheet. Examples of preservatives include parabens, chlorobutanol and sorbic acid, and examples of absorption promoters include surfactants such as alcohols, fatty acids and salts thereof, alcohol amines, alkyl ethers, glycerides or alkyl glycosides, and water-soluble solvents such as dimethylsulfoxide, dimethylformamide or pyrrolidones.

A liquid or lotion preparation can be prepared by using a general-purpose base such as water (purified water or distilled water), lower alcohol such as ethanol, polyvalent alcohol such as glycerin and surfactant such as polyoxyethylene (60) hydrogenated castor oil.

An ophthalmic preparation can be prepared by suitably selecting and using an isotonic agent such as sodium chloride, potassium chloride or concentrated glycerin, a buffering agent such as sodium phosphate, sodium acetate or epsilon-aminocaproic acid, a surfactant such as polyoxyethylene sorbitan monooleate, polyoxyl 40 stearate or polyoxyethylene hydrogenated castor oil, a stabilizer such as sodium citrate or sodium edetate, and a preservative such as benzalkonium chloride or an paraben as necessary, and although pH is only required to be within the range allowed by ophthalmological preparations, normally it is preferably within the range of 4.0 to 8.0. The ophthalmic preparation may be in the form of a solution or suspension.

An injection preparation can be prepared by selecting and using an isotonic agent such as sodium chloride, a buffering agent such as sodium phosphate, a surfactant such as polyoxyethylene sorbitan monooleate, and a thickener such as methyl cellulose as necessary.

An insert preparation can be prepared by crushing and mixing a biodegradable polymer such as hydroxypropyl cellulose, hydroxypropyl methyl cellulose, carboxyvinyl polymer or polyacrylic acid with the present compound, followed by compression molding the resulting powder. An additive such as an excipient, binder, stabilizer or pH adjuster can be used in the insert preparation as necessary.

A preparation for intraocular implant can be prepared by using a biodegradable polymer such as polylactic acid, polyglycolic acid, lactic acid-glycolic acid copolymer or hydroxypropyl cellulose. An additive such as an excipient, binder, stabilizer or pH adjuster can be used in the preparation for intraocular implant as necessary.

Although varying according to the administration route and administration form, the concentration of the nandrolone, ester thereof or salt thereof, or methenolone, ester thereof or salt thereof contained in the present dry eye therapeutic agent is preferably, for example, 0.000001 to 50 %(w/v). In the case of selecting a drug form such as a cream or ointment preparation suitable for administration to eyelid skin, the concentration is 0.00001 to 40 %(w/w), preferably 0.0001 to 30 %(w/w), more preferably 0.0005 to 20 %(w/w), even more preferably 0.1 to 10 %(w/w), and most preferably 1 to 5 %(w/w). Furthermore, in the present invention, in the case of containing a salt of nandrolone or an ester thereof, or a salt of methenolone, an ester thereof, these values are calculated by converting to free nandrolone or an ester thereof, or free methenolone or an ester thereof.

In addition, in the case of selecting a patch for the drug form suitable for administration to eyelid skin, the amount of nandrolone, an ester thereof or a salt thereof, or methenolone, an ester thereof or a salt thereof administered to the eyelid skin of a dry eye patient is, for example, 0.00005 mg to 100 mg, preferably 0.0005 mg to 50 mg, and most preferably 0.0025 mg to 25 mg.

In addition, in the case of selecting an administration route other than administration to eyelid skin, such as an ophthalmic preparation administered to the keratoconjunctiva of the eye, the amount of nandrolone, an ester thereof or a salt thereof, or methenolone, an ester thereof or a salt thereof administered to the keratoconjunctiva of the eye of a dry eye patient is, for example, 0.0001 to 5% (w/v), preferably 0.001 to 3% (w/v), more preferably 0.01 to 2% (w/v), and even more preferably 0.1 to 1% (w/v).

Although usage of the present dry eye therapeutic agent can be suitably modified corresponding to such factors as the drug form, severity of symptoms, age and body weight of the patient to receive administration, or the discretion of a physician, in the case of selecting administration to eyelid skin for the administration route, the present dry eye therapeutic agent can be administered to eyelid skin at a frequency of, for example, twice per day, once per day, once every 2 days, once every 3 days, once every 4 days, once every 5 days, once every 6 days or once every 7 days, and is preferably administered to eyelid skin once per day. Furthermore, in the present invention, "administration" includes the application of a patch preparation to an administration site. Thus, in the case of, for example, selecting a patch preparation for the administration form, "administering to eyelid skin at a frequency of once per day" means "applying to eyelid skin at a frequency of once per day".

The aforementioned detailed explanation of the dry eye therapeutic agent of the present invention is also applied to the aspects of the present invention indicated below.

One aspect of the present invention is a pharmaceutical composition for treating dry eye comprising nandrolone, an ester thereof or a salt thereof, or methenolone, an ester thereof or a salt thereof, and a pharmaceutically acceptable additive.

One aspect of the present invention is a use of nandrolone, an ester thereof or a salt thereof, or methenolone, an ester thereof or a salt thereof for the manufacture of a medicament for treating dry eye.

One aspect of the present invention is nandrolone, an ester thereof or a salt thereof, or methenolone, an ester thereof or a salt thereof, for use in the treatment dry eye.

One aspect of the present invention is a use of nandrolone, an ester thereof or salt thereof, or methenolone, an ester thereof or a salt thereof, for the treatment of dry eye.

One aspect of the present invention is a method for the treatment of dry eye, comprising administering a therapeutically effective amount of nandrolone, an ester thereof or a salt thereof, or methenolone, an ester thereof or a salt thereof, to a subject.

Although the following indicates the results of pharmacological studies and preparation examples, these examples are intended to facilitate understanding of the present invention, and are not intended to limit the scope thereof.

### [Examples]

### [Pharmacological Study (1)]

A study was conducted in rabbits as to whether or not the administration of nandrolone decanoate and methenolone acetate to eyelid skin increases the amount of TL in tears.

### (Drug Preparation Method)

A cream containing 1% (w/v) of nandrolone decanoate, a cream containing 1% (w/v) of methenolone acetate and a base were prepared in the manner of the compositions indicated in Table 1.

**[Table 1]**

| | Cream containing 1% (w/v) nandrolone decanoate | Cream containing 1% (w/v) methenolone acetate | Base |
|---|---|---|---|
| Nandrolone decanoate | 1.0g | - | - |
| Methenolone acetate | - | 1.0g | - |
| Miglyol 812 | 30mL | 30mL | 30mL |
| Cremophor EL | 5mL | 5mL | 5mL |
| Concentrated glycerin | 0.8mL | 0.8mL | 0.8mL |
| Polycarbophil | 0.6g | 0.6g | 0.6g |
| Methyl paraben | 0.1g | 0.1g | 0.1g |
| Propyl paraben | 0.03g | 0.03g | 0.03g |
| Ethanol | As suitable | As suitable | As suitable |
| Sodium hydroxide | As suitable | As suitable | As suitable |
| Purified water | As suitable | As suitable | As suitable |
| Total | 100mL | 100mL | 100mL |

### (Study Method and Drug Administration Method)

Rabbits (Japanese White, females, purchased from Kitayama Labes Co., Ltd.) were used in the study. The lower lacrimal gland and nictitating membrane of the rabbits were preliminarily excised under general anesthesia coupled with daily oral administration of ketoconazole starting four weeks prior to drug application for the purpose of inhibiting the production of androgen. Fur around the eyelids was shaved using an electric hair clipper prior to the start of application, followed by fitting the rabbits with Elizabethan collars and applying cream containing 1% (w/v) nandrolone decanoate or cream containing 1% (w/v) methenolone acetate to the eyelid skin of one eye of rabbits in the drug dose groups once per day (40 µL per application), while leaving the other eye untreated (n=4). The base was applied in the same manner to rabbits of a control group (n=4).

### (Evaluation Method)

Schirmer test paper (Showa Yakuhin Kako Co., Ltd.) was inserted into the conjunctival sac of the lower eyelid before application and two weeks after application to collect tears, followed by subjecting each of the collected tear specimens to slab gel electrophoresis. Following electrophoresis, the gel was stained with Coomassie Brilliant Blue stain, all protein bands, including the TL band at a molecular weight of about 17 kilodaltons, were detected, and the percentage of TL (%TL) in the total amount of protein present in the tears was calculated by image analysis of the resulting gel. The difference between the %TL before application and the %TL after application was determined for each individual and expressed as an increase in %TL (Δ%TL).

### (Results)

As shown in Table 2, since considerable increases in TL were observed in the drug administration groups in comparison with the control group, administration of nandrolone decanoate or methenolone acetate to eyelid skin was indicated to increase the amount of TL present in tears.

**[Table 2]**

| Group | Δ%TL |
|---|---|
| Nandrolone decanoate administration group | 4.30 ± 0.473 |
| Methenolone acetate administration group | 2.70 ± 0.314 |
| Control group | 0.28 ± 0.295 |

Values indicate mean ± standard error.

### (Discussion)

On the basis of the above results, nandrolone or an ester thereof or methenolone or an ester thereof was discovered to increase TL levels, and was indicated to demonstrate a therapeutic effect on dry eye. In particular, since administration of nandrolone decanoate to eyelid skin increased the amount of TL more than that of methenolone acetate, it was suggested to demonstrate a potent therapeutic effect on dry eye.

### [Pharmacokinetics Study (1)]

A study was conducted for changes over time of methenolone concentration in the lacrimal glands after administration of methenolone acetate to eyelid skin.

### (Drug Preparation Method)

0.4 g of methenolone acetate were added to castor oil followed by bringing to a volume of 20 mL and dissolving therein to prepare an ointment containing 2% (w/v) of methenolone acetate.

### (Study Method and Drug Administration Method)

Rabbits (Japanese White, males, purchased from Kitayama Labes Co., Ltd.) were used in the study. Fur of the right upper eyelid was shaved by using an electric hair clipper under general anesthesia on the day before drug administration followed by fitting the rabbits with Elizabethan collars prior to administration. A single administration of ointment containing 2 %(w/v) methenolone acetate (50 µL) was applied to the skin of the right upper eyelid.

### (Evaluation Method)

The rabbits were euthanized at 2, 4, 8 and 24 hours after administration and the right upper lacrimal gland tissue was excised (from 3 animals at each time). The lacrimal gland tissue was homogenized in methanol and the concentration of methenolone (active form of methenolone acetate) in the centrifuged supernatant was measured by LC-MS/MS. The concentration of methenolone in the lacrimal gland tissue was calculated from the wet weight of the tissue measured prior to homogenization and the concentration of methenolone in the measured specimen.

### (Results)

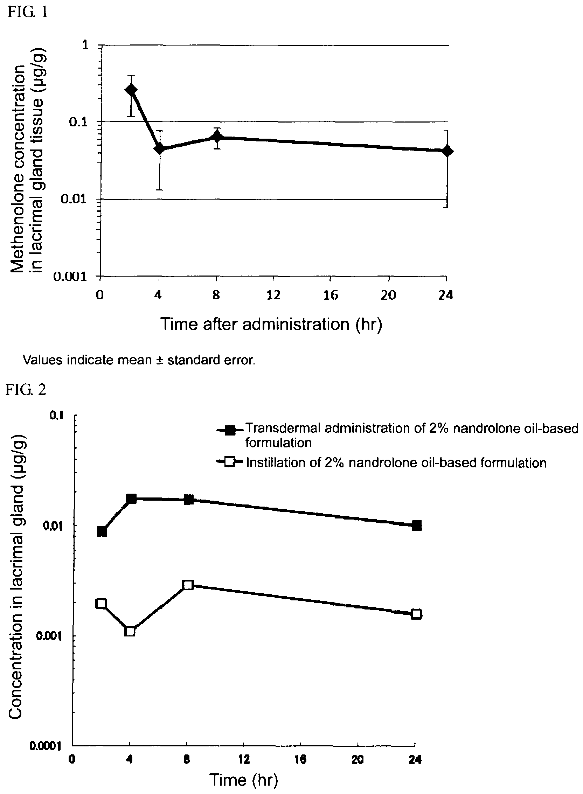

As shown in FIG. 1, the concentration of methenolone in the lacrimal gland after administration of methenolone acetate to eyelid skin hardly changed from 4 hours to 24 hours after administration, and was observed to be maintained over a long period of time.

### (Discussion)

As has been described above, in the case of having administered methenolone acetate to eyelid skin, since the concentration of methenolone in lacrimal gland tissue sustained for at least 24 hours, methenolone or an ester thereof was indicated to continuously demonstrate a therapeutic effect on dry eye by administering to eyelid skin at a frequency of once per day.

### [Pharmacokinetics Study (2)]

A study was conducted for change over time of nandrolone concentration in the lacrimal glands after administration of nandrolone decanoate to eyelid skin, or instillation thereof.

### (Drug Preparation Method)

0.4 g of nandrolone decanoate were added to castor oil followed by bringing to a volume of 20 mL and dissolving therein to prepare an ointment containing 2% (w/v) of nandrolone decanoate. In addition, a preparation of the same composition was used as an ophthalmic solution containing nandrolone decanoate.

### (Study Method and Drug Administration Method)

Rabbits (Japanese White, males, purchased from Kitayama Labes Co., Ltd.) were used in the study. Fur of the right upper eyelid was shaved by using an electric hair clipper under general anesthesia on the day before drug administration followed by fitting the rabbits with Elizabethan collars prior to administration. A single administration of ointment containing 2% (w/v) nandrolone decanoate (50 µL) was applied to the skin of the right upper eyelid in one of the groups (n=4).

On the other hand, a single administration of ophthalmic solution containing 2% (w/v) nandrolone decanoate (50 µL) was dropped into the right eye in the other group (n=4).

### (Evaluation Method)

The rabbits were euthanized at 2, 4, 8 and 24 hours after administration and the right upper lacrimal gland tissue was excised (from 3 animals at each time). The lacrimal gland tissue was homogenized in methanol and the concentration of nandrolone (active form of nandrolone decanoate) in the centrifuged supernatant was measured by LC-MS/MS. The concentration of nandrolone in the lacrimal gland tissue was calculated from the wet weight of the tissue measured prior to homogenization and the concentration of nandrolone in the measured specimen.

### (Results)

As shown in FIG. 2, the concentration of nandrolone in the lacrimal gland after administration of nandrolone decanoate to eyelid skin was observed to be maintained at a high concentration from 4 hours to at least 24 hours after administration. On the other hand, although the concentration of nandrolone in the lacrimal gland after instillation of nandrolone decanoate was observed to be maintained for at least 24 hours after administration, the concentration was clearly determined to be much lower than in the case of administration to eyelid skin.

### (Discussion)

On the basis of the above, in the case of having administered nandrolone decanoate to eyelid skin, dry eye therapeutic effects were indicated to obtain more potent than the case of the administration thereof by instillation. Moreover, since the concentration of nandrolone in lacrimal gland tissue in the case of having administered nandrolone decanoate to eyelid skin is maintained at a high concentration for at least 24 hours, nandrolone or an ester thereof was indicated to continuously demonstrate a therapeutic effect on dry eye by administering to eyelid skin at a frequency of once per day.

On the other hand, when compared with the results of Pharmacokinetics Study (1) described above, the concentration of nandrolone in lacrimal gland tissue after administration of nandrolone decanoate to eyelid skin was indicated to be somewhat lower than the concentration of methenolone in lacrimal gland tissue after administration of methenolone acetate to eyelid skin.

### [Pharmacological Study (2)]

A comparative study of the action of increasing the expression level of lipophilin CL2 (L-CL2) gene by both nandrolone decanoate and methenolone acetate was conducted by allowing both drugs to act on lacrimal gland tissue cells. Furthermore, lipophilins are proteins that are synthesized in the lacrimal gland and are observed to be present in large amounts in the tears of humans and rabbits.

### (Drug Preparation Method)

Each drug was dissolved in DMSO followed by diluting with DMEM/F-12 (D/F medium) to a final DMSO concentration of 0.1%.

### (Study Method)

Lacrimal glands harvested from rabbits (Japanese White, females, purchased from Kitayama Labes Co., Ltd.) were sliced into thin sections followed by repeatedly subjecting to shaking treatment in HBSS containing EDTA and shaking treatment in DMEM containing collagenase, hyaluronidase and DNase I to digest the lacrimal gland tissue. After removing undigested tissue, isolated lacrimal gland cells were prepared by carrying out density gradient centrifugation with 2% to 4% Ficoll. The resulting cells were suspended in D/F medium and disseminated in a 96-well plate coated with type I collagen. After culturing the cells for 2 days at 37°C and 5% CO₂ + 95% gaseous phase, the medium was replaced with D/F medium containing each concentration of drug and further cultured for 2 days.

### (Evaluation Method)

Quantitative PCR was carried out by using primers designed by respectively joining to cDNA synthesized from the drug-treated lacrimal gland cells using the CellAmp Direct RNA Prep Kit and PrimeScript RT MasterMix (both manufactured by Takara Bio, Inc.), the QuantiFast SYBR Green PCR Kit (Qiagen Corp.), Rabbit L-CL2 gene (Accession No.: NM_001082095) and Rabbit GAPDH gene (Accession No.: NM_001082253). The expression level of L-CL2 gene of each of the drug-treated cells was corrected with expression of each GAPDH gene and expressed as the relative ratio to the expression level of cells treated with base (D/F medium containing 0.1% DMSO).

### (Results)

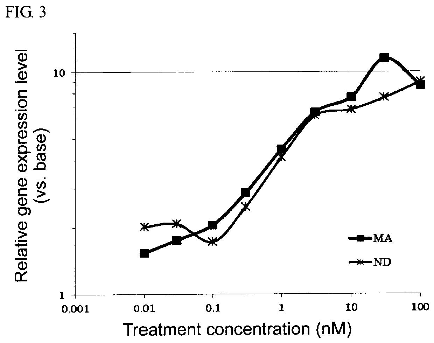

As shown in FIG. 3, nandrolone decanoate (ND) and methenolone acetate (MA) were indicated to demonstrate an action that increases expression of L-CL2 gene in lacrimal gland cells, and the potency of that action was nearly of the same degree.

### (Discussion)

When lacrimal gland tissue cells were treated by nandrolone decanoate or methenolone acetate, both demonstrated effect of increased expression of L-CL2 gene, and the activating effect on the lacrimal gland were roughly equal. Despite this, as previously described in the aforementioned Pharmacological Study (1), administration of nandrolone decanoate to eyelid skin resulted in a more potent effect of enhancing TL than administration of an equal concentration of methenolone acetate to eyelid skin. Moreover, as described in the aforementioned Pharmacokinetics Studies (1) and (2), the amount of the active form that migrates to the lacrimal gland after administration to eyelid skin is smaller for nandrolone decanoate than methenolone acetate, and in comparison with methenolone acetate, nandrolone decanoate therefore has a more potent increasing action on tear proteins, and especially on TL.

### [Preparation Examples]

Although the following provides a more detailed explanation of the therapeutic agent of the present invention by indicating preparation examples, the present invention is not limited thereto.

### Formulation Example 1: Cream preparation(2% (w/v))

In 100 mL:

| | |
|---|---|
| Methenolone acetate | 2.0 g |
| Miglyol 812 | 30 mL |
| Cremophor EL | 5 mL |
| Concentrated glycerin | 0.8 mL |
| Polycarbophil | 0.6 g |
| Methyl paraben | 0.1 g |
| Propyl paraben | 0.03 g |
| Ethanol | As suitable |
| Sodium hydroxide | As suitable |

| | |
|---|---|
| Purified water | As suitable |

Methenolone acetate is added to a mixture of Miglyol 812, Cremophor EL and concentrated glycerin and dissolved, therein followed by sequentially adding polycarbophil, methyl paraben, propyl paraben and purified water while stirring. Sodium hydroxide is added so that the pH reaches about 7, followed by stirring well to prepare a cream. In addition, creams having a concentration of methenolone acetate of, for example, 1% (w/v), 3% (w/v) or 5% (w/v) can also be prepared by suitably changing the amounts of methenolone acetate and other additives added.

### Formulation Example 2: Cream preparation (1% (w/v))

In 100 mL:

| | |
|---|---|
| Nandrolone decanoate | 1.0 g |
| Miglyol 812 | 30 mL |
| Cremophor EL | 5 mL |
| Concentrated glycerin | 0.8 mL |
| Polycarbophil | 0.6 g |
| Methyl paraben | 0.1 g |
| Propyl paraben | 0.03 g |
| Ethanol | As suitable |
| Sodium hydroxide | As suitable |
| Purified water | As suitable |

Nandrolone decanoate is added to a mixture of Miglyol 812, Cremophor EL and concentrated glycerin and dissolved therein, followed by sequentially adding polycarbophil, methyl paraben, propyl paraben and purified water while stirring. Sodium hydroxide is added so that the pH reaches about of 7 followed by stirring well to prepare a cream. In addition, creams having a concentration of nandrolone decanoate of, for example, 2% (w/v), 3% (w/v) or 5% (w/v) can also be prepared by suitably changing the amounts of nandrolone decanoate and other additives added.

### Formulation Example 3: Ointment preparation(2% (w/w))

In 100 g:

| | |
|---|---|
| Nandrolone decanoate | 2.0 g |
| Liquid paraffin | 39.2 g |
| White vaseline | 58.8 g |

Nandrolone decanoate is added to homogenously melted white vaseline and liquid paraffin followed by mixing well and cooling gradually to prepare an ointment. In addition, ointments having a concentration of nandrolone decanoate of, for example, 1% (w/w) or 3% (w/w) can also be prepared by suitably changing the amounts of nandrolone decanoate and other additives added.

### Formulation Example 4: Patch preparation (1 mg)

In 10 g of plaster:

| | |
|---|---|
| Nandrolone decanoate | 0.1 g |
| Polyethylene glycol 400 | 1.1 g |
| Polyvinylpyrrolidone | 1.9 g |
| Ethanol | 6.9 g |

After dissolving nandrolone decanoate in a mixture of polyethylene glycol 400, polyvinylpyrrolidone and ethanol and spreading the mixture out in the form of a tape, the tape was dried by heating. After pressing onto a support, the tape was covered with a release film and suitably cut to prepare a patch. In addition, patches having different concentrations of nandrolone decanoate can also be prepared by suitably changing the amounts of nandrolone decanoate and other additives added.

### Formulation Example 5: Ophthalmic preparation (0.1% (w/v))

In 100 mL:

| | |
|---|---|
| Nandrolone decanoate | 0.1 g |
| Polysorbate 80 | 0.5 g |
| Sodium chloride | 0.9 g |
| Dilute hydrochloric acid | As suitable |
| Sodium hydroxide | As suitable |
| Purified water | As suitable |

Nandrolone decanoate, Polysorbate 80 and sodium chloride are added to water and dissolved therein followed by adjusting the pH to about 7 using dilute hydrochloric acid and sodium hydroxide to prepare an ophthalmic preparation. In addition, ophthalmic preparation having a concentration of nandrolone decanoate of, for example, 0.2% (w/v), 0.3% (w/v) or 0.5% (w/v) can also prepared by suitably changing the amounts of nandrolone decanoate and other additives added. In addition, the ophthalmic preparation may be in the form of a solution or suspension.

### INDUSTRIAL APPLICABILITY

Nandrolone, an ester thereof or a salt thereof, or methenolone acetate, an ester thereof or a salt thereof, demonstrates a therapeutic effect on dry eye.

## Claims

1. A dry eye therapeutic agent comprising nandrolone, an ester thereof or a salt thereof, or methenolone, an ester thereof or a salt thereof as an active ingredient.

2. The dry eye therapeutic agent according to claim 1, which is **characterized by** being administered to eyelid skin.

3. The dry eye therapeutic agent according to claim 1 or 2, which comprises nandrolone decanoate as an active ingredient.

4. The dry eye therapeutic agent according to claim 1 or 2, which comprises methenolone acetate as an active ingredient.

5. The dry eye therapeutic agent according to claim 3 or 4, which comprises nandrolone decanoate or methenolone acetate at a concentration of 0.1 to 10 %(w/w) as an active ingredient.

6. The dry eye therapeutic agent according to claim 3 or 4, which comprises nandrolone decanoate or methenolone acetate at a concentration of 1 to 5 %(w/w) as an active ingredient.

7. The dry eye therapeutic agent according to any one of claims 1 to 6, which is **characterized by** being administered once a day to eyelid skin.

8. The dry eye therapeutic agent according to any one of claims 1 to 7, wherein the administration form is an ointment or cream.

9. The dry eye therapeutic agent according to any one of claims 1 to 7, wherein the administration form is a patch.

10. A composition for secretory promotion of secretions from lacrimal glands, comprising nandrolone, an ester thereof or a salt thereof, or methenolone, an ester thereof or a salt thereof as an active ingredient.

11. The composition according to claim 10, which is **characterized by** being administered to eyelid skin.

12. The composition according to claim 10 or 11, which comprises nandrolone decanoate as an active ingredient.

13. The composition according to claim 10 or 11, which comprises methenolone acetate as an active ingredient.

14. The composition according to claim 12 or 13, which comprises nandrolone decanoate or methenolone acetate at a concentration of 0.1 to 10 %(w/w) as an active ingredient.

15. The composition according to claim 12 or 13, which comprises nandrolone decanoate or methenolone acetate at a concentration of 1 to 5 %(w/w) as an active ingredient.

16. The composition according to any one of claims 10 to 15, which is **characterized by** being administered once a day to eyelid skin.

17. The composition according to any one of claims 10 to 16, wherein the administration form is an ointment or cream.

18. The composition according to any one of claims 10 to 16, wherein the administration form is a patch.

19. A pharmaceutical composition for treating dry eye comprising nandrolone, an ester thereof or a salt thereof, or methenolone, an ester thereof or a salt thereof, and a pharmaceutically acceptable additive.

20. The pharmaceutical composition according to claim 19, which is **characterized by** being administered to eyelid skin.

21. The pharmaceutical composition according to claim 19 or 20, which comprises nandrolone decanoate as an active ingredient.

22. The pharmaceutical composition according to claim 19 or 20, which comprises methenolone acetate as an active ingredient.

23. The pharmaceutical composition according to claim 21 or 22, which comprises nandrolone decanoate or methenolone acetate at a concentration of 0.1 to 10 %(w/w) as an active ingredient.

24. The pharmaceutical composition according to claim 21 or 22, which comprises nandrolone decanoate or methenolone acetate at a concentration of 1 to 5 %(w/w) as an active ingredient.

25. The pharmaceutical composition according to any one of claims 19 to 24, which is **characterized by** being administered once a day to eyelid skin.

26. The pharmaceutical composition according to any one of claims 19 to 25, wherein the administration form is an ointment or cream.

27. The pharmaceutical composition according to any one of claims 19 to 25, wherein the administration form is a patch.

28. A use of nandrolone, an ester thereof or a salt thereof, or methenolone, an ester thereof or a salt thereof, for the manufacture of a medicament for treating dry eye.

29. The use according to claim 28, **characterized in that** the medicament is administered to eyelid skin.

30. The use according to claim 28 or 29, wherein the medicament contains nandrolone decanoate as an active ingredient.

31. The use according to claim 28 or 29, wherein the medicament contains methenolone acetate as an active ingredient.

32. The use according to claim 30 or 31, wherein the medicament contains nandrolone decanoate or methenolone acetate at a concentration of 0.1 to 10 %(w/w) as an active ingredient.

33. The use according to claim 30 or 31, wherein the medicament contains nandrolone decanoate or methenolone acetate at a concentration of 1 to 5 %(w/w) as an active ingredient.

34. The use according to any one of claims 28 to 33, **characterized in that** the medicament is administered once a day to eyelid skin.

35. The use according to any one of claims 28 to 34, wherein the administration form of the medicament is an ointment or cream.

36. The use according to any one of claims 28 to 34, wherein the administration form of the medicament is a patch.

37. Nandrolone, an ester thereof or a salt thereof, or methenolone, an ester thereof or a salt thereof, for use in the treatment of dry eye.

38. The nandrolone, an ester thereof or a salt thereof, or methenolone, an ester thereof or a salt thereof, according to claim 37, which is **characterized by** being administered to eyelid skin.

39. The nandrolone, an ester thereof or a salt thereof according to claim 37 or 38, which is **characterized by** being administered in the form of nandrolone decanoate.

40. The methenolone, an ester thereof or a salt thereof according to claim 37 or 38, which is **characterized by** being administered in the form of methenolone acetate.

41. The nandrolone, an ester thereof or a salt thereof, or methenolone, an ester thereof or a salt thereof, according to claim 39 or 40, **characterized in that** nandrolone decanoate or methenolone acetate is administered at a concentration of 0.1 to 10 %(w/w).

42. The nandrolone, an ester thereof or a salt thereof, or methenolone, an ester thereof or a salt thereof, according to claim 39 or 40, **characterized in that** nandrolone decanoate or methenolone acetate is administered at a concentration of 1 to 5 %(w/w).

43. The nandrolone, an ester thereof or a salt thereof, or methenolone, an ester thereof or a salt thereof, according to any one of claims 37 to 42, which is **characterized by** being administered once a day to eyelid skin.

44. The nandrolone, an ester thereof or a salt thereof, or methenolone, an ester thereof or a salt thereof, according to any one of claims 37 to 43, **characterized in that** the administration form is an ointment or cream.

45. The nandrolone, an ester thereof or a salt thereof, or methenolone, an ester thereof or a salt thereof, according to any one of claims 37 to 43, **characterized in that** the administration form is a patch.

46. A method for the treatment of dry eye, comprising administering a therapeutically effective amount of nandrolone, an ester thereof or a salt thereof, or methenolone, an ester thereof or a salt thereof, to a subject.

47. The method for the treatment according to claim 46, **characterized in that** the administration is to eyelid skin.

48. The method for the treatment according to claim 46 or 47, **characterized in that** nandrolone decanoate is administered.

49. The method for the treatment according to claim 46 or 47, **characterized in that** methenolone acetate is administered.

50. The method for the treatment according to claim 48 or 49, **characterized in that** nandrolone decanoate or methenolone acetate is administered at a concentration of 0.1 to 10 %(w/w).

51. The method for the treatment according to claim 48 or 49, **characterized in that** nandrolone decanoate or methenolone acetate is administered at a concentration of 1 to 5 %(w/w).

52. The method for the treatment according to any one of claims 46 to 51, **characterized in that** the administration is once a day to eyelid skin.

53. The method for the treatment according to any one of claims 46 to 52, wherein the administration form is an ointment or cream.

54. The method for the treatment according to any one of claims 46 to 52, wherein the administration form is a patch.

55. A method for secretory promotion of secretions from lacrimal glands, comprising administering an effective amount of nandrolone, an ester thereof or a salt thereof, or methenolone, an ester thereof or a salt thereof, to a subject.

56. The method according to claim 55, **characterized in that** the administration is to eyelid skin.

57. The method according to claim 55 or 56, **characterized in that** nandrolone decanoate is administered.

58. The method according to claim 55 or 56, **characterized in that** methenolone acetate is administered.

59. The method according to claim 57 or 58, **characterized in that** nandrolone decanoate or methenolone acetate is administered at a concentration of 0.1 to 10 %(w/w).

60. The method according to claim 57 or 58, **characterized in that** nandrolone decanoate or methenolone acetate is administered at a concentration of 1 to 5 %(w/w).

61. The method according to any one of claims 55 to 60, **characterized in that** the administration is once a day to eyelid skin.

62. The method according to any one of claims 55 to 61, wherein the administration form is an ointment or cream.

63. The method according to any one of claims 55 to 61, wherein the administration form is a patch.
